# FASCICULE DE BREVET EUROPEEN

(11) **EP 1 485 153 B1**
(45) Date de publication et mention de la délivrance du brevet: **30.01.2008**
(21) Numéro de dépôt: 03722694.1
(22) Date de dépôt: 06.03.2003
(51) Int. Cl.: A61M 5/50, A61M 5/32

(54) **DISPOSITIF DE PROTECTION D AIGUILLE DESTINE A UNE SERINGUE, ET DISPOSITIF D INJECTION COMPRENANT UNE SERINGUE ET CE DISP OSITIF DE PROTECTION**
NADELSCHUTZVORRICHTUNG FÜR EINE SPRITZE SOWIE INJEKTIONSVORRICHTUNG BESTEHEND AUS EINER SPRITZE UND DIESER SCHUTZVORRICHTUNG
NEEDLE PROTECTION DEVICE FOR A SYRINGE AND AN INJECTION DEVICE COMPRISING A SYRINGE AND SAID PROTECTION DEVICE

(30) Priorité: 18.03.2002 FR 0203338
(43) Date de publication de la demande: 15.12.2004
(73) Titulaire: SEDAT, 69540 Irigny (Rhône) (FR)
(72) Inventeur: PESSIN, Olivier, F-69290 GREZIEU LA VARENNE (FR)
(74) Mandataire: Blot, Philippe Robert Emile
(86) Numéro de dépôt international: PCT/FR2003/000722
(87) Numéro de publication internationale: WO 2003/077977

(56) Documents cités:
- EP-A- 1 066 848
- WO-A-99/17823
- US-A- 5 346 480
- US-A- 5 573 513
- US-A- 6 033 386
- US-A1- 2001 031 949

## Description

La présente invention concerne un dispositif de protection d'aiguille destiné à une seringue qui comprend une aiguille, un corps tubulaire à l'extrémité distale duquel est fixée l'aiguille, et un piston d'injection monté à coulissement dans le corps, ce dispositif comportant un protecteur d'aiguille sensiblement coaxial au corps de la seringue ; un support de protecteur solidaire dudit corps ou pourvu de moyens de solidarisation audit corps ; un ressort disposé en appui entre le support et le protecteur, le support et le protecteur étant mobiles l'un par rapport à l'autre entre une position rétractée du protecteur dans laquelle l'aiguille de la seringue est dégagée et le ressort est verrouillé dans un état comprimé, et une position déployée du protecteur dans laquelle l'aiguille est disposée à l'intérieur du protecteur et le ressort est, au moins partiellement, détendu ; et des moyens de déverrouillage du ressort.

L'invention s'applique notamment au domaine des seringues d'injection pré-remplies à usage unique, destinées en particulier aux injections intramusculaires ou sous-cutanées (voir p.ex. US 5 573 513).

Ces dispositifs sont destinés à limiter au maximum pour l'utilisateur les risques de piqûres accidentelles post-injection puisqu'une fois l'injection du contenu de la seringue réalisée, l'aiguille de la seringue est retirée du patient et le protecteur est amené en position déployée tout autour de l'aiguille.

Pour garantir une plus grande sûreté d'utilisation, on a proposé d'équiper ces dispositifs de moyens élastiques de déplacement du protecteur autour de l'aiguille de la seringue. Ainsi, le document US 5 591 138 décrit un dispositif du type précité, qui peut être utilisé d'une des deux façons suivantes : une fois l'injection réalisée par une main, l'utilisateur actionne par son autre main les moyens de déverrouillage pour dégager le ressort de compression et permettre le déploiement du protecteur autour de l'aiguille. Ou bien l'aiguille est enfoncée suffisamment profondément dans la peau du patient pour provoquer le déverrouillage du ressort, ce dernier ne pouvant pas pour autant se détendre en raison de l'appui du protecteur contre la peau du patient ; dans ce cas, le ressort se détend lorsque l'ensemble du dispositif est écarté du patient.

Ce genre de dispositif nécessite donc pour obtenir le déploiement voulu du protecteur, soit une manipulation à deux mains, soit l'appui du protecteur sur la peau du patient. Cette manipulation ne garantit ni une grande commodité d'utilisation pour le praticien, ni un grand confort pour le patient. Ces dispositifs restent donc peu employés car d'utilisation trop éloignée de l'utilisation courante d'une seringue d'injection. De plus, une fois le ressort déverrouillé, l'énergie de décompression du ressort se libère souvent violemment, sans contrôle possible par l'utilisateur, ce qui provoque une certaine instabilité du dispositif dans la main de l'utilisateur. Par ailleurs, le blocage rigide du protecteur en position déployée, notamment pour éviter toute ré-utilisation du dispositif, n'est possible qu'en manipulant à deux mains le dispositif.

Le but de la présente invention est de proposer un dispositif de protection qui permette de déployer le protecteur par la même main que celle qui tient la seringue, avec un geste de déclenchement simple sans appui nécessaire du protecteur contre le patient, et un contrôle possible du déplacement du protecteur par l'utilisateur.

A cet effet, l'invention a pour objet un dispositif de protection du type défini plus haut et pour lequel le ressort présente, à l'état verrouillé, une force prédéterminée supérieure à l'effort de poussée nécessaire pour déplacer le piston de la seringue sur sensiblement toute sa course d'injection, et les moyens de déverrouillage comportent une collerette d'appui digital, solidaire du protecteur, et adaptée pour, d'une part, lors du coulissement du piston pour l'injection, transmettre au ressort une force de compression inférieure à ladite force prédéterminée, et d'autre part, lorsque le piston est en fin de course d'injection, transmettre au ressort une force de compression supérieure à ladite force prédéterminée.

Suivant d'autres caractéristiques de ce dispositif, prises isolément ou selon toutes les combinaisons techniquement possibles :
- l'effort de poussée est au moins égal à la somme de l'effort d'injection, et des efforts de décollement et de glissement du piston à l'intérieur du corps de la seringue ;
- la collerette est située à l'extrémité proximale du protecteur ;
- la longueur du protecteur est sensiblement égale à celle du corps de la seringue ;
- pour une seringue dont le corps est solidairement pourvu à son extrémité proximale d'un col, les moyens de solidarisation du support de protecteur au corps de la seringue comportent des moyens de fixation dudit support sur le col de la seringue,
- lesdits moyens de fixation comportent au moins un crochet de clipsage du col de la seringue ;
- le support et le protecteur sont montés en rotation l'un par rapport à l'autre entre la position rétractée du protecteur et une position de déverrouillage du ressort ;
- les moyens de déverrouillage comportent d'une part au moins une première rainure ménagée dans l'un du support et du protecteur, et au moins en partie inclinée par rapport à la direction axiale du corps de la seringue, et d'autre part au moins un pion solidaire de l'autre du support et du protecteur, et reçu dans ladite première rainure, la rainure et le pion coopérant pour guider en rotation le support et le protecteur l'un par rapport à l'autre entre la position rétractée du protecteur et la position de déverrouillage du ressort ;
- au moins une deuxième rainure de réception du pion, communiquant avec la première rainure, est ménagée dans celui du support ou du protecteur dans lequel est ménagée la première rainure, ladite deuxième rainure s'étendant sensiblement dans la direction axiale du corps de la seringue de façon à pouvoir guider en translation le support et le protecteur l'un par rapport à l'autre entre la position de déverrouillage du ressort et la position déployée du protecteur ;
- le fond distal de la deuxième rainure forme une butée de blocage axial, suivant le sens de déploiement du protecteur, pour le pion associé ;
- il comporte des moyens de blocage en position déployée du protecteur ;
- lesdits moyens de blocage comportent d'une part au moins une languette solidaire du support, et d'autre part au moins un crochet déformable, solidaire du protecteur, chaque languette et chaque crochet ménagent respectivement deux surfaces d'appui sensiblement conjuguées, adaptées pour déformer radialement le crochet correspondant lorsque le support et le protecteur passent de la position rétractée à la position déployée du protecteur, et chaque languette et chaque crochet ménagent respectivement deux surfaces de butée axiale sensiblement conjuguée, adaptée pour bloquer le support et le protecteur l'un par rapport à l'autre dans la position déployée du protecteur ;
- il comporte des moyens de contrôle de première utilisation du dispositif ; et
- lesdits moyens de contrôle comportent d'une part un capuchon amovible adapté pour être disposé autour de l'aiguille de la seringue avant utilisation, et d'autre part des languettes déformables ménagées à l'extrémité distale du protecteur et délimitant une ouverture de passage dont le diamètre est, lorsque lesdites languettes ne sont pas déformées, inférieur au diamètre maximal du capuchon.

L'invention a en outre pour objet un dispositif d'injection comportant d'une part une seringue qui comprend une aiguille, un corps tubulaire à l'extrémité distale duquel est fixée l'aiguille, et un piston d'injection monté à coulissement dans le corps, et d'autre part un dispositif de protection d'aiguille qui comprend à la fois un protecteur d'aiguille sensiblement coaxial au corps de la seringue, un support de protecteur solidaire dudit corps ou pourvu de moyens de solidarisation au corps de la seringue, un ressort disposé en appui entre le support et le protecteur, le support et le protecteur étant mobiles l'un par rapport à l'autre entre une position rétractée du protecteur dans laquelle l'aiguille de la seringue est dégagée et le ressort est verrouillé dans un état comprimé, et une position déployée dans laquelle l'aiguille est disposée à l'intérieur du protecteur et le ressort est, au moins partiellement, détendu, et des moyens de déverrouillage du ressort, dans lequel le dispositif de protection d'aiguille est tel que défini ci-dessus.

L'invention sera mieux comprise à la lecture de la description qui va suivre, donnée uniquement à titre d'exemple et faite en se référant aux dessins sur lesquels :
- la figure 1 est une vue éclatée en perspective d'un dispositif d'injection selon l'invention avant assemblage ;
- la figure 2 est une vue en élévation du dispositif de protection appartenant au dispositif d'injection de la figure 1, en position rétractée ;
- la figure 3 est une vue en coupe selon le plan III-III indiqué sur la figure 2 ;
- la figure 4 est une vue de dessus selon la flèche IV indiquée sur la figure 2 ;
- la figure 5 est une vue en coupe selon le plan indiqué V-V sur la figure 4;
- la figure 6A est une vue suivant le même plan de coupe que celui de la figure 5, du dispositif d'injection selon l'invention avant utilisation ;
- la figure 6B est une vue de dessus selon la flèche VI indiquée sur la figure 6A ;
- la figure 7 est une vue analogue à celle de la figure 6A, le dispositif d'injection étant en cours d'utilisation ;
- la figure 8 est une vue suivant le même plan de coupe que celui de la figure 3, du dispositif d'injection en fin de d'utilisation ;
- la figure 9 est une vue identique à celle de la figure 8, la seringue du dispositif d'injection n'étant pas représentée ;
- la figure 10 est une vue en coupe selon le plan X-X indiquée sur la figure 8 ; et
- les figures 11 à 13 illustrent le dispositif d'injection en position déployée, la figure 11 étant une vue analogue à celle de la figure 8, la figure 12 étant une vue en coupe selon le plan XII-XII indiqué sur la figure 11 et la figure 13 est une vue identique à celle de la figure 11, la seringue n'étant pas représentée.

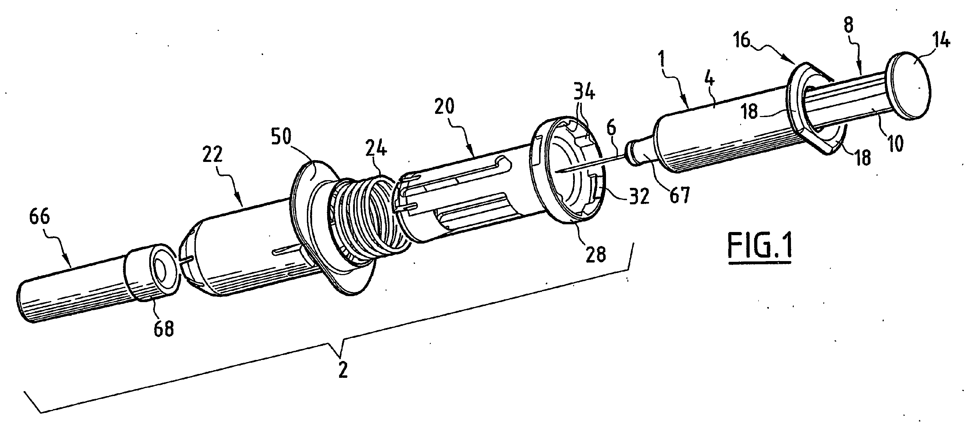

Sur la figure 1 sont représentés en perspective une seringue 1 et un ensemble de protection 2. Dans toute la suite, les termes « proximal » et « arrière » sont synonymes, tout comme les termes « distal » et « avant ».

La seringue 1 est une seringue en verre de forme standard, destinée à un usage unique. Elle contient un liquide à injecter, de façon intramusculaire ou sous-cutanée, à un patient. Elle comporte à cet effet un corps 4, une aiguille 6 solidaire de l'extrémité distale du corps 4 et un piston 8. Ce piston comporte classiquement une tige 10 pourvue à son extrémité distale d'un poussoir 12 non visible sur la figure 1 et d'une tête d'appui 14 sur laquelle est destiné à s'appuyer le pouce de la main du praticien.

Le corps 4 de la seringue comporte dans sa partie proximale un col 16 délimitant circonférentiellement deux oreilles 18 diamétralement opposées, destinées normalement, notamment en l'absence de l'ensemble 2, à former des surfaces d'appui pour l'index et le majeur du praticien lors de la manipulation de la seringue et de l'injection du liquide s'y trouvant.

L'ensemble de protection 2, d'axe général X-X, comporte essentiellement, comme représenté sur la figure 1 :
- un support 20 de forme générale tubulaire ;
- un manchon protecteur 22 disposé coaxialement au support 20 et de diamètre supérieur à celui du support, et
- un ressort 24.

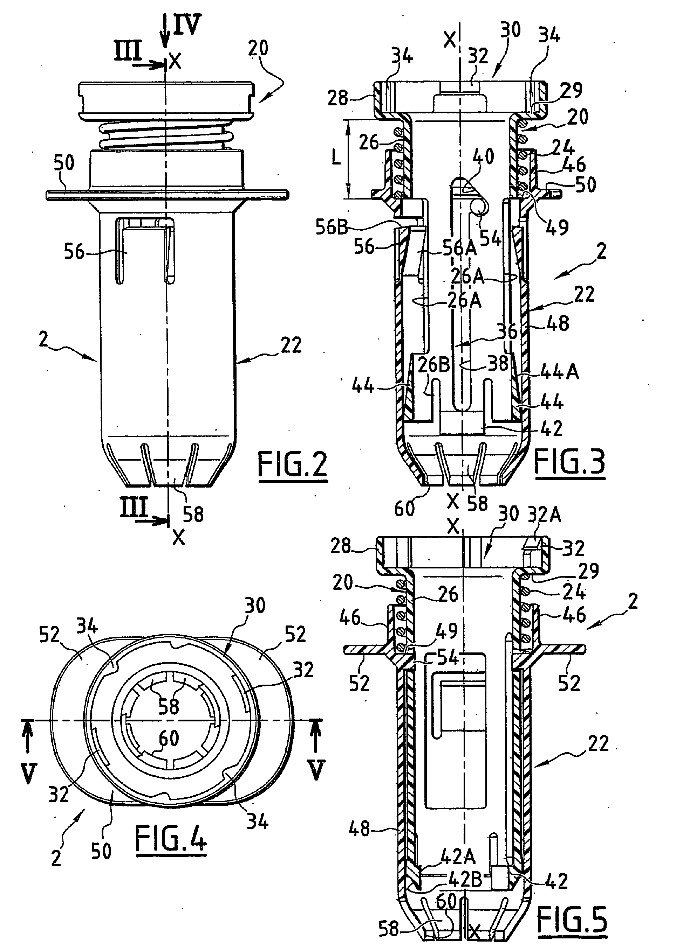

Ces trois éléments vont successivement être détaillés ci-dessous, en regard des figures 2 à 4.

Le support 20 comporte un tronçon principal 26, sensiblement cylindrique et de diamètre interne sensiblement égal au diamètre externe du corps 4 de la seringue 1. Ce tronçon 26 se prolonge à son extrémité proximale par un tronçon secondaire 28 de plus grands diamètres intérieur et extérieur que ceux du tronçon principal 26, en formant un épaulement radial 29.

Le tronçon proximal 28 est pourvu de moyens 30 de fixation sur le col de seringue 16. Plus précisément, ces moyens 30 comportent d'une part une paire de crochets déformables 32 diamétralement opposées, également visibles sur la figure 1. Chacun de ces crochets ménage une surface de rampe 32A sensiblement tronconique s'évasant vers l'extrémité libre du tronçon 28, destinée à être repoussée élastiquement par les oreilles 18 du col de seringue 16 lors de la fixation du support 20 sur la seringue 1. Les crochets 32 sont distants de l'épaulement 29 d'une distance sensiblement égale à l'épaisseur des oreilles 18. Les crochets 32 forment ainsi un moyen de clipsage du col de seringue 16.

Les moyens de fixation 30 comportent d'autre part des excroissances 34 (figures 1, 4 et 6B) qui s'étendent en saillie radialement vers l'intérieur du tronçon 28. Ces excroissances sont adaptées pour former d'une part des moyens d'indexage angulaire, à 180° près, du col 16 de la seringue, et d'autre part des butées de blocage en rotation pour ce col 16 lorsque celui-ci est retenu axialement entre les crochets 32 et l'épaulement 29.

Deux rainures traversantes 36 sont ménagées l'une en face de l'autre dans le tronçon principal 26. Chaque rainure est constituée d'une première partie rectiligne 38 qui s'étend sensiblement suivant l'axe X-X du support 20 sur une longueur supérieure à celle de l'aiguille 6, et d'une seconde partie rectiligne 40 qui s'étend de manière inclinée par rapport au même axe X-X. La partie inclinée 40 débouche dans la partie d'extrémité proximale de la partie de rainure principale 38, en formant un V dont la pointe est dirigée du côté proximal de l'ensemble 2.

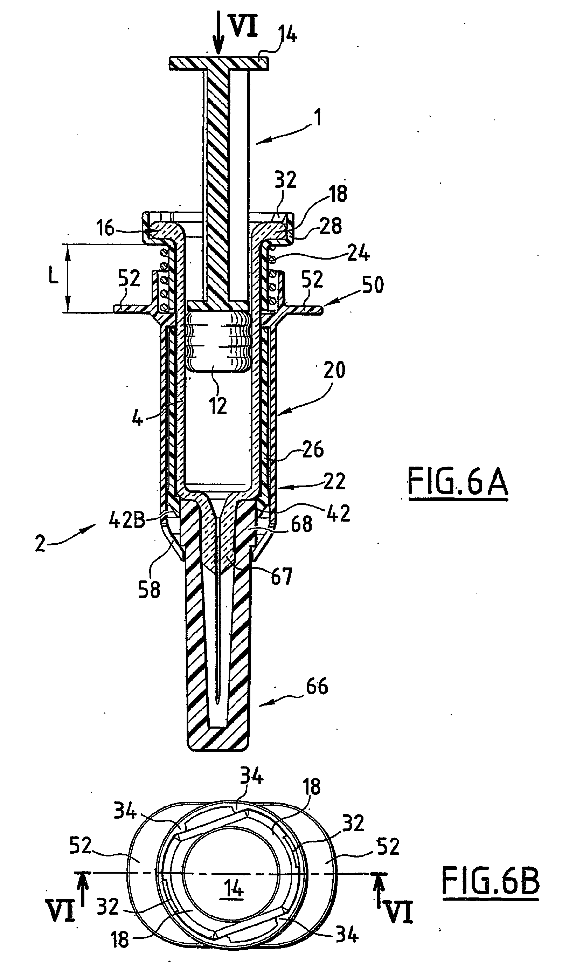

Le tronçon principal 24 comporte à son extrémité distale une première paire de languettes 42 diamétralement opposées, situées chacune dans le prolongement des rainures 36 (figure 5). Ces languettes 42 présentent une face interne 42A de forme sensiblement cylindrique et une face externe 42B sensiblement tronconique, divergente vers l'arrière.

Le tronçon principal 24 comporte une seconde paire de languettes 44 diamétralement opposées, situées entre les languettes 42 en suivant la circonférence de l'extrémité distale de ce tronçon. Elles présentent une surface externe 44A sensiblement tronconique, divergente vers l'avant, et une surface distale 44B sensiblement plane.

A l'aplomb proximal de ces languettes 44, des évidements longitudinaux 26A sont ménagés dans le tronçon principal 26 (figure 3).

Par ailleurs, de part et d'autre des languettes 42, des fentes axiales 26B sont prévues à partir de l'extrémité distale du support, de sorte que, avant insertion du corps 4 de la seringue 1 à l'intérieur du support 20, ces languettes 42 sont radialement déformables, notamment vers l'intérieur.

Le manchon protecteur 22 est de longueur sensiblement égale à celle du corps 4 de la seringue 1. Il est constitué de deux tronçons cylindriques 46 et 48, le tronçon proximal 46 étant de diamètre légèrement supérieur à celui du tronçon principal 48. Ces deux tronçons se raccordent en formant un épaulement radial 49.

Le manchon 22 comporte solidairement, dans sa partie proximale, une collerette extérieure 50 sous forme de deux oreilles 52 diamétralement opposées (figure 4).

Egalement dans sa partie proximale, mais à l'intérieur du manchon protecteur 22, deux tétons 54 diamétralement opposés sont solidaires du manchon (figure 5). Ces deux tétons sont adaptés pour être reçus et guidés dans respectivement les deux rainures 36 du support 20, le support et le manchon étant ainsi mobiles l'un par rapport à l'autre en translation suivant leur axe commun et en rotation limitée autour du même axe lorsque les pions 54 se trouvent dans les parties de rainures 40.

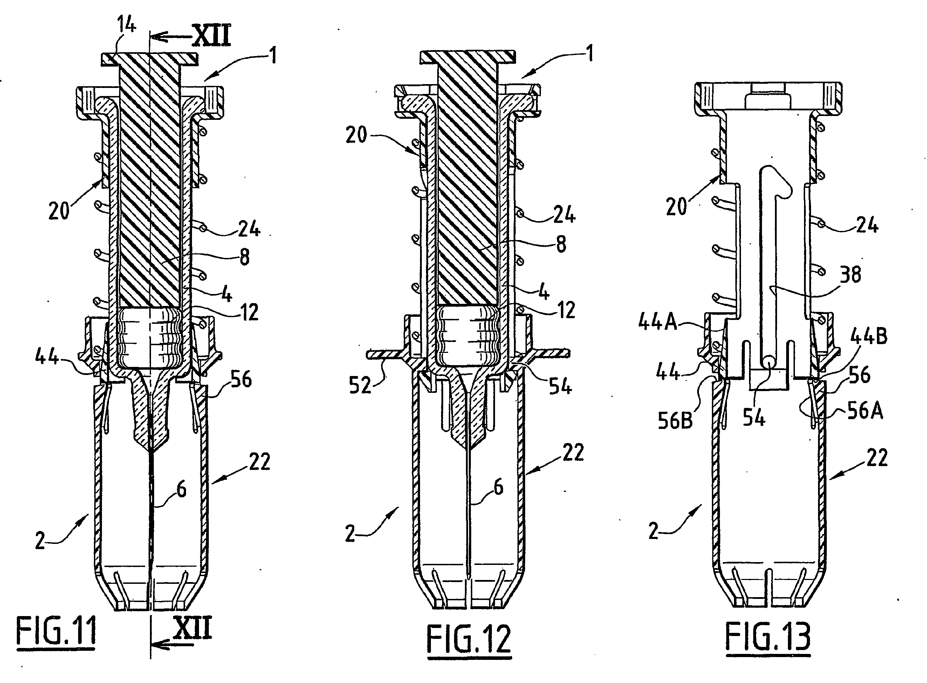

Plus précisément, le support 20 et le manchon 22 sont mobiles entre une position rétractée du manchon, dans laquelle l'essentiel du manchon recouvre l'essentiel du support et les tétons 54 sont situés à l'extrémité distale de chacune des parties de rainures inclinées 40, comme représenté sur les figures 2 à 7, et une position déployée du manchon dans laquelle celui-ci s'étend axialement en saillie du support et les tétons sont situés à l'extrémité distale de la partie de rainure longitudinale 38, comme représenté sur les figures 11 à 13.

Lorsque la seringue 1 est fixée sur l'ensemble 2, ces positions extrêmes correspondent respectivement à une configuration d'injection dans laquelle l'aiguille 6 de la seringue 1 est dégagée et destinée à être insérée dans un patient, et à une configuration de protection dans laquelle cette aiguille est entourée du manchon protecteur 22.

La partie proximale du manchon 22 comporte en outre intérieurement une paire de crochets déformables 56 diamétralement opposés. Chacun de ces crochets présente d'une part une surface interne 56A de forme sensiblement tronconique divergente vers l'avant, complémentaire de celle des surfaces internes 44A des languettes 44 du support 20, et une surface proximale 56B sensiblement plane (figure 3).

Dans la position rétractée du manchon, ces crochets 56 s'étendent à l'intérieur des évidements 26A ménagés dans le support 20. Dans la position déployée du manchon, comme représenté sur la figure 11, les faces 56B des crochets 56 sont en butée axiale contre les languettes 44, les crochets et languettes formant de la sorte un ensemble de blocage rigide en position déployée.

Le manchon 22 est par ailleurs pourvu à son extrémité distale d'une couronne de languettes déformables 58, dont les bords distaux forment une ouverture 60 sensiblement circulaire, de diamètre inférieur au diamètre intérieur du tronçon principal 26 du support 20.

Le ressort 24 est un ressort spiralé, disposé entre le manchon protecteur 22 et le support de protecteur 20. Plus précisément, le ressort est logé entre l'épaulement 29 du support 20 et l'épaulement 49 du manchon 22. Dans la position rétractée du manchon, le ressort 24 est dans un état comprimé, possédant ainsi une énergie de décompression liée à la raideur du ressort et à la différence entre la longueur du ressort à l'état au repos et sa longueur, notée L sur les figures 2 à 7, à l'état comprimé. Cela revient à dire que le ressort 24 présente un seuil de force de compression supplémentaire qui correspond à la force minimale nécessaire pour comprimer davantage le ressort à partir de son état comprimé initial des figures 2 à 7. La raideur du ressort et/ou la longueur de compression initiale L sont choisies de telle sorte que ce seuil de force soit supérieur à l'effort de poussée nécessaire pour déplacer le piston 8 de la seringue 1 sur toute sa course d'injection. Plus précisément, la force du ressort à l'état verrouillé est supérieure à la somme de l'effort d'injection, c'est-à-dire d'évacuation du liquide à l'extérieur de l'aiguille 6 de la seringue 1, et des efforts de décollement et de glissement du poussoir 12 à l'intérieur du corps 4 de la seringue.

En option, l'ensemble de protection 2 comporte en outre un capuchon 66 de forme générale tubulaire, représenté sur les figures 1 et 6A.

Le capuchon 66 est adapté pour entourer l'aiguille 6 avant utilisation de la seringue 1. Ce capuchon est fermé à une de ses extrémités et son extrémité opposée est formée par une bague annulaire 68 de diamètre extérieur adapté pour à la fois être conjugué de la surface 42A des crochets 42 du support 20 et être supérieur au diamètre de l'ouverture 60 formée par les languettes 58 du manchon protecteur 22. La face intérieure de cette bague 68 est destinée à adhérer sur la base en verre 67 du corps de seringue 4 où est fixée l'aiguille 6, notamment pour garantir une certaine étanchéité aux bactéries.

Le fonctionnement du dispositif d'injection selon l'invention est le suivant :

D'une part, le dispositif de protection 2 est assemblé dans sa configuration rétractée, c'est-à-dire celle des figures 2 à 7. Le manchon protecteur 22 est pour cela enfilé autour du support 20 à partir de l'extrémité distale du support, en disposant entre eux le ressort 24. Plus précisément, on déplace axialement vers l'arrière le manchon par rapport au support, tout en déformant radialement vers l'extérieur les crochets 56 au moyen d'un outil adapté, et ce au moins jusqu'à ce qu'ils atteignent axialement la partie avant des évidements longitudinaux 26A. Puis toujours en déplaçant le manchon vers l'arrière, les pions 54 sont appliqués contre les surfaces externes 42B des languettes 42, en déformant ces dernières vers l'intérieur, jusqu'à ce que les pions soient reçus dans les parties de rainures 38. Le déplacement du protecteur 22 vers l'arrière se poursuit ensuite jusqu'à ce que les pions 54 soient reçus dans les parties de rainures inclinées 40, en faisant pivoter l'un par rapport à l'autre le support et le protecteur. Le protecteur se retrouve ainsi en position rétractée.

D'autre part, la seringue en verre 1 est préremplie d'un liquide à injecter dans un patient. Cette seringue est équipée du capuchon 66 qui enserre la base 67 du corps de seringue 4.

La seringue et le capuchon sont ensuite insérés à l'intérieur de l'ensemble 2 pour former le dispositif d'injection, tel que représenté sur les figures 6A et 6B. Plus précisément, le corps 4 de la seringue est déplacé sensiblement axialement à l'intérieur du support 20 jusqu'à ce que le col de seringue 16, indexé par les excroissances 34, s'enclipse à l'intérieur du tronçon proximal 28, en déformant radialement vers l'extérieur les crochets 32. Les oreilles 18 du col de seringue 16 sont alors retenues axialement par les crochets 32, tandis que les excroissances périphériques 34 retiennent le col de seringue, et donc la seringue, en rotation par rapport au support 20.

De plus, dans la mesure où le col de seringue 16 est totalement enclipsé à l'intérieur du tronçon 28, il ne peut plus remplir son rôle habituel de formation de surface d'appui pour l'index et le majeur du praticien. Cette fonction d'appui est réalisée par la collerette 50 solidaire du manchon 22. En effet, dans la mesure où la longueur du manchon protecteur 22 est sensiblement égale à celle du corps de seringue 4 et/ou où la collerette 50 est ménagée au niveau de l'extrémité proximale de ce manchon, le praticien peut alors manipuler la seringue en faisant reposer son pouce sur la tête d'appui 14 du piston 8 comme à l'accoutumée, et en faisant reposer son index et son majeur sur les faces des oreilles 52 dirigées vers l'aiguille 6.

Par ailleurs, lorsque la seringue 1 est fixée sur le support de protecteur 20 comme représenté sur les figures 6A et 6B, le capuchon 66 s'étend en saillie à l'extérieur du manchon protecteur 22.

Lorsque le praticien est prêt à réaliser l'injection du liquide contenu dans la seringue, il retire le capuchon 66 en le tirant axialement vers l'avant. La bague 68 franchit alors l'ouverture 60 en déformant les languettes 58. Une fois le capuchon 66 retiré, les languettes 58 reprennent leur position initiale. Le trou de passage 60 à l'extrémité distale du manchon, de diamètre plus petit que le diamètre extérieur du capuchon 68, empêche alors la remise en place du capuchon autour de l'aiguille. La coopération du capuchon 66 et des languettes 58 forment ainsi un moyen de contrôle de la première utilisation du dispositif d'injection.

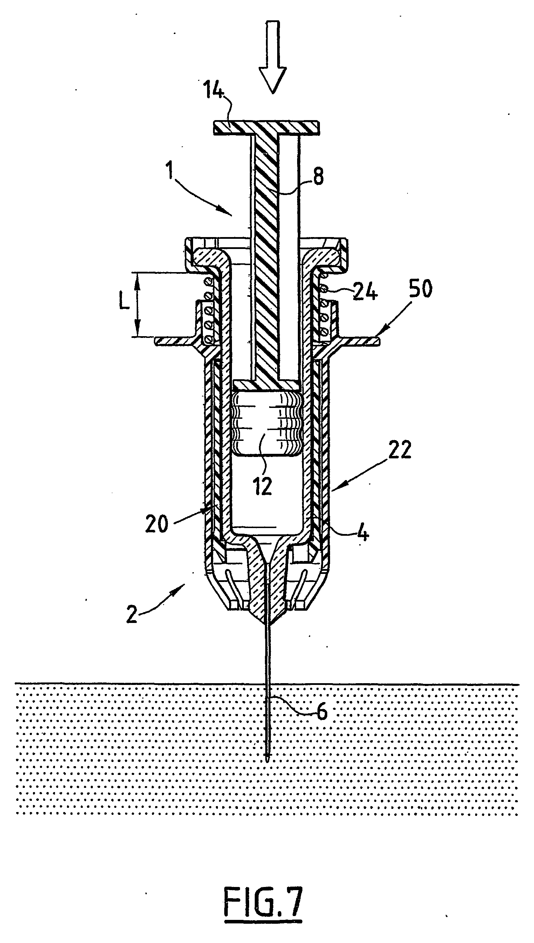

Le praticien insère ensuite l'aiguille 6 dans un patient. Il injecte le liquide contenu dans la seringue en exerçant une poussée sur la tête d'appui 14 du piston 8, son index et son majeur demeurant en contact avec les faces dirigées vers l'aiguille des oreilles 52. Durant l'injection, aucun mouvement ne se produit entre le support de protecteur 20 et le manchon protecteur 22, le ressort 24 demeurant comprimé avec une longueur L, comme représenté sur la figure 7.

L'injection se poursuit jusqu'à ce que le poussoir 12 du piston 8 parvienne en fin de course d'injection.

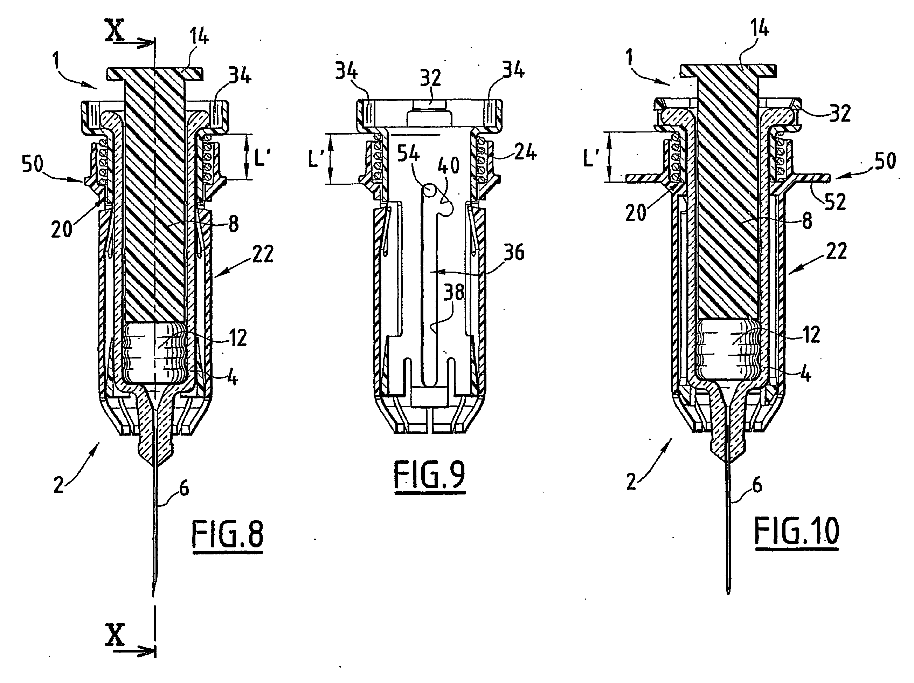

Le praticien retire alors l'aiguille du patient. Pour déclencher l'ensemble de protection 2, le praticien exerce une pression supplémentaire sur la tige de piston 8. Cette pression doit être supérieure à la force prédéterminée produite par le ressort 24 à l'état verrouillé, de sorte que ce ressort est davantage comprimé et passe de sa longueur L à une longueur L' plus petite, comme représenté sur les figures 8 à 10. Pour ce faire, en raisonnant à support de seringue immobile, le manchon protecteur 24 se déplace axialement vers l'extrémité proximale du support 20. Le praticien réalise ce mouvement en exerçant une pression correspondante au moyen de son index et de son majeur sur les oreilles 52 de la collerette 50 du manchon 22. Cette pression entraîne, de façon combinée au mouvement de translation, la rotation du manchon protecteur 22 autour du support de seringue 20, les tétons 54 étant guidés par les parties de rainure inclinées 40. Ce mouvement de rotation se poursuit jusqu'à ce que les tétons atteignent l'extrémité proximale de cette partie de rainure 40, c'est-à-dire l'extrémité proximale de la partie de rainure longitudinale 38, comme visible sur la figure 9. Le dispositif 2 est alors en position de déverrouillage du ressort 24.

Le praticien relâche ensuite la pression qu'il exerçait jusqu'alors sur la collerette 50, permettant au ressort 24 de se détendre jusqu'à un état de repos. Les pions 54 se déplacent en translation à l'intérieur de la partie de rainure longitudinale 38, jusqu'à l'extrémité distale de celle-ci comme représenté sur la figure 13. Le mouvement de translation du manchon 22 par rapport au support 20 est contrôlable par le praticien, si ce dernier relâche progressivement la retenue digitale qu'il exerce la collerette 50. Lorsque les pions 54 sont arrivés à l'extrémité distale de la rainure 36 (figure 13), le protecteur est dans sa position déployée.

Par ailleurs, lorsque le manchon 22 est en mouvement de translation par rapport au support 20, les crochets 56 empruntent les évidements longitudinaux 26A du support, jusqu'à ce qu'ils glissent le long des languettes distales 44 du support 22, par coopération de leurs surfaces complémentaires 56A et 44A.

En position déployée du protecteur, les languettes 44 maintiennent en position les crochets 56 par coopération des surfaces 56B et 44B, de sorte que le manchon protecteur 22 ne peut pas être ramené dans sa position initiale. De même, le manchon 44 ne peut pas être facilement arraché du support 20 puisque les pions 54 sont en butée contre le fond distal de la partie de rainure longitudinale 38 (figure 13), les languettes 42 formant ce fond étant radialement maintenues entre le corps de la seringue 1 et le manchon 22.

Le dispositif d'injection selon l'invention est ainsi simple d'utilisation, tout en permettant au praticien de contrôler le mouvement de recouvrement de l'aiguille par le manchon protecteur. Le nombre de pièces dont est constitué l'ensemble de protection 2 représenté est réduit à trois.

Le dispositif selon l'invention est adaptable à différents types de seringues, tant au niveau des formes qu'à celui des volumes. Ce dispositif présente donc l'avantage de ne pas remettre en cause la forme générale des seringues utilisées et par conséquent n'entraîne aucune modification des procédés industriels de remplissage de ces seringues.

Diverses variantes du dispositif selon l'invention sont envisageables :
- à la différence de l'exemple de réalisation décrit ci-dessus, les pions 54 et/ou la collerette 50 du manchon protecteur 22 peuvent être rapportés sur le manchon 22 et non réalisés d'un seul tenant avec celui-ci ;
- à l'inverse du dispositif décrit, les tétons 54 peuvent être réalisés sur la surface externe du support de protecteur 20 et la rainure de guidage 36 ménagée dans le manchon protecteur 22 ;
- le support 20 peut être réalisé d'une seule pièce avec le corps de seringue 4 ; et/ou
- les excroissances 34 des moyens 30 de fixation du support 20 sur le col de seringue 16 peuvent être supprimées et éventuellement remplacées par un ou plusieurs crochets analogues aux crochets 32 décrits ci-dessus, l'ensemble de ces crochets retenant axialement le col 16 par rapport au support 20 pour toute position angulaire relative entre la seringue 1 et le support ; dans ce cas, la seringue 1 est libre de tourner à l'intérieur du support 20, ce qui assure en contre-partie une plus grande facilité de fixation de l'ensemble de protection 2 sur la seringue (absence d'indexage).

## Revendications

1. Dispositif de protection d'aiguille destiné à une seringue (1) qui comprend une aiguille (6), un corps tubulaire (4) à l'extrémité distale duquel est fixée l'aiguille (6), et un piston d'injection (8) monté à coulissement dans le corps (4), ce dispositif comprenant un protecteur d'aiguille (22), sensiblement coaxial au corps (4) de la seringue (1) ; un support de protecteur (20) solidaire dudit corps (4) ou pourvu de moyens (30) de solidarisation audit corps (4) ; un ressort (24) disposé en appui entre le support (20) et le protecteur (22), le support et le protecteur étant mobiles l'un par rapport à l'autre entre une position rétractée du protecteur dans laquelle l'aiguille (6) de la seringue (1) est dégagée et le ressort (24) est verrouillé dans un état comprimé, et une position déployée dans laquelle l'aiguille est disposée à l'intérieur du protecteur (22) et le ressort est, au moins partiellement, détendu ; et des moyens de déverrouillage du ressort (24), **caractérisé en ce que** le ressort (24) présente, à l'état verrouillé, une force prédéterminée supérieure à l'effort de poussée nécessaire pour déplacer le piston (8) de la seringue (1) sur sensiblement toute sa course d'injection, et **en ce que** les moyens de déverrouillage comportent une collerette (50) d'appui digital, solidaire du protecteur (22), et adaptée pour, d'une part, lors du coulissement du piston (8) pour l'injection, transmettre au ressort (24) une force de compression inférieure à ladite force prédéterminée, et d'autre part, lorsque le piston (8) est en fin de course d'injection, transmettre au ressort (24) une force de compression supérieure à ladite force prédéterminée.

2. Dispositif suivant la revendication 1, **caractérisé en ce que** l'effort de poussée est au moins égal à la somme de l'effort d'injection, et des efforts de décollement et de glissement du piston (8) à l'intérieur du corps (4) de la seringue (1).

3. Dispositif suivant l'une des revendications 1 ou 2, **caractérisé en ce que** la collerette (50) est située à l'extrémité proximale du protecteur (22).

4. Dispositif suivant l'une quelconque des revendications précédentes, **caractérisé en ce que** la longueur du protecteur (22) est sensiblement égale à celle du corps (4) de la seringue (1).

5. Dispositif suivant l'une quelconque des revendications précédentes, **caractérisé en ce que**, pour une seringue (1) dont le corps (4) est solidairement pourvu à son extrémité proximale d'un col (16), les moyens de solidarisation du support de protecteur (20) au corps (4) de la seringue (1) comportent des moyens (30) de fixation dudit support sur le col (16) de la seringue (1).

6. Dispositif suivant la revendication 5, **caractérisé en ce que** lesdits moyens de fixation (30) comportent au moins un crochet (32) de clipsage du col (16) de la seringue (1).

7. Dispositif suivant l'une quelconque des revendications précédentes, **caractérisé en ce que** le support (20) et le protecteur (22) sont montés en rotation l'un par rapport à l'autre entre la position rétractée du protecteur et une position de déverrouillage du ressort (24).

8. Dispositif suivant la revendication 7, **caractérisé en ce que** les moyens de déverrouillage comportent d'une part au moins une première rainure (40) ménagée dans l'un du support (20) et du protecteur (22), et au moins en partie inclinée par rapport à la direction axiale du corps (4) de la seringue (1), et d'autre part au moins un pion (54) solidaire de l'autre du support (20) et du protecteur (22), et reçu dans ladite première rainure (40), la rainure et le pion coopérant pour guider en rotation le support et le protecteur l'un par rapport à l'autre entre la position rétractée du protecteur et la position de déverrouillage du ressort (24).

9. Dispositif suivant la revendication 8, **caractérisé en ce qu'**au moins une deuxième rainure (38) de réception du pion (54), communiquant avec la première rainure (40), est ménagée dans celui du support (20) ou du protecteur (22) dans lequel est ménagée la première rainure (40), ladite deuxième rainure (38) s'étendant sensiblement dans la direction axiale du corps (4) de la seringue (1) de façon à pouvoir guider en translation le support (20) et le protecteur (22) l'un par rapport à l'autre entre la position de déverrouillage du ressort (24) et la position déployée du protecteur.

10. Dispositif suivant la revendication 9, **caractérisé en ce que** le fond distal de la deuxième rainure (38) forme une butée de blocage axial, suivant le sens de déploiement du protecteur (22), pour le pion associé (54).

11. Dispositif suivant l'une quelconque des revendications précédentes, **caractérisé en ce qu'**il comporte des moyens (44, 56) de blocage en position déployée du protecteur (22).

12. Dispositif suivant la revendication 11, **caractérisé en ce que** lesdits moyens de blocage comportent d'une part au moins une languette (44) solidaire du support (20), et d'autre part au moins un crochet déformable (56), solidaire du protecteur (22), **en ce que** chaque languette (44) et chaque crochet (56) ménagent respectivement deux surfaces d'appui sensiblement conjuguées, adaptées pour déformer radialement le crochet (56) correspondant lorsque le support (20) et le protecteur (22) passent de la position rétractée à la position déployée du protecteur, et ce que chaque languette (44) et chaque crochet (56) ménagent respectivement deux surfaces (44B, 56B) de butée axiale sensiblement conjuguée, adaptée pour bloquer le support (20) et le protecteur (22) l'un par rapport à l'autre dans la position déployée du protecteur.

13. Dispositif suivant l'une quelconque des revendications précédentes, **caractérisé en ce qu'**il comporte des moyens de contrôle de première utilisation du dispositif (2).

14. Dispositif suivant la revendication 13, **caractérisé en ce que** lesdits moyens de contrôle comportent d'une part un capuchon amovible (66) adapté pour être disposé autour de l'aiguille (6) de la seringue (1) avant utilisation, et d'autre part des languettes déformables (58) ménagées à l'extrémité distale du protecteur (22) et délimitant une ouverture de passage (60) dont le diamètre est, lorsque lesdites languettes (58) ne sont pas déformées, inférieur au diamètre maximal du capuchon (66).

15. Dispositif d'injection comprenant d'une part une seringue (1) qui comprend une aiguille (6), un corps tubulaire (4) à l'extrémité distale duquel est fixée l'aiguille (6), et un piston d'injection (8) monté à coulissement dans le corps (4), et d'autre part un dispositif (2) de protection d'aiguille qui comprend un protecteur d'aiguille (22), sensiblement coaxial au corps (4) de la seringue (1), un support de protecteur (20) solidaire dudit corps (4) ou pourvu de moyens (30) de solidarisation audit corps (4), un ressort (24) disposé en appui entre le support (20) et le protecteur (22), le support et le protecteur étant mobiles l'un par rapport à l'autre entre une position rétractée du protecteur dans laquelle l'aiguille (6) de la seringue (1) est dégagée et le ressort (24) est verrouillé dans un état comprimé, et une position déployée dans laquelle l'aiguille est disposée à l'intérieur du protecteur (22) et le ressort est, au moins partiellement, détendu, et des moyens de déverrouillage du ressort (24), **caractérisé en ce que** le dispositif de protection d'aiguille (2) est conforme à l'une quelconque des revendications 1 à 14.

## Claims

1. Device for protection of needles designed for syringes (1) comprised of a needle (6), a tubular body (4) at the distal end of which the needle (6) is fixed, and an injection piston (8) fitted so that it slides inside the body (4), where this device is comprised of a needle protector (22), effectively coaxial with the body (4) of the syringe (1); a protector support (20) firmly attached to the said body (4) or provided with means (30) for being firmly attached to the said body (4); a spring (24) arranged so that it is supported between the support (20) and the protector (22), with the support and the protector capable of movement in relation to each other between a retracted position of the protector, in which the needle (6) of the syringe (1) is exposed and the spring (24) is locked is a compressed state, and a deployed position in which the needle is arranged inside the protector (22) and the spring is, at least partly, released; and means of locking the spring (24), **characterised by** the fact that the spring (24) exerts, in the locked state, a predetermined force which is greater than the thrust force required to displace the piston (8) of the syringe (1) effectively over its entire injection travel, and by the fact that the means of unlocking include a finger support flange (50), firmly attached to the protector (22) and designed so that, on the one hand, during the sliding motion of the piston (8) for injection, it transmits to the spring (24) a compression force which is less than the said predetermined force, and on the other hand, when the piston (8) is at the end of injection travel, it transmits to the spring (24) a compression force which is greater than the predetermined force.

2. Device according to claim 1, **characterised by** the fact that the thrust force is at least equal to the sum of the injection force, and the forces for releasing and sliding the piston (8) inside the body (4) of the syringe (1).

3. Device according to any of claims 1 to 2, **characterised by** the fact that the flange (50) is located at the proximal end of the protector (22),

4. Device according to any of the preceding claims whatsoever, **characterised by** the fact that the length of the protector (22) is effectively equal to that of the body (4) of the syringe (1).

5. Device according to any of the preceding claims whatsoever, **characterised by** the fact that for a syringe (1) whose body (4), at its proximal end, has a firmly attached collar (16), the means for firmly attaching the protector support (20) to the body (4) of the syringe (1) include means (30) for fastening the said support on the collar (16) of the syringe (1).

6. Device as described in claim 5, **characterised by** the fact that the said means of fastening (30) include at least one hook (32) for clipping the collar (16) of the syringe (1).

7. Device according to any of the preceding claims whatsoever, **characterised by** the fact that the support (20) and the protector (22) are fitted so that they can rotate in relation to each other between the protector retracted position and a position in which the spring is unlocked (24).

8. Device according to claim 7, **characterised by** the fact that the means of unlocking include on the one hand at least one first groove (40) made in one of the support (20) and the protector(22), and at least in the inclined part relative to the axial direction of the body (4) of the syringe (1), and on the other hand at least one pin (54) firmly attached to the other of the support (20) and the protector (22), which is received in the said first groove (40), with the groove and the pin fitting together to guide the rotation of the support and the protector relative to each other between the retracted position of the protector and the unlocked position of the spring (24).

9. Device according to claim 8, **characterised by** the fact that at least one second groove (38) for receiving the pin (54), which connects with the first groove (40), is formed in that of the support (20) or of the protector (22) in which the first groove is made (4), where the said second groove (38) effectively extends in the axial direction of the body (4) of the syringe (1) so that it can effectively guide the translation movement of the support (20) and the protector (22) relative to each other between the unlocking position of the spring (24) and the deployed position of the protector.

10. Device according to claim 9, **characterised by** the fact that the distal end of the second groove (38) forms an axial blocking end-stop, along the direction of deployment of the protector (22), for the associated pin (54).

11. Device according to any of the preceding claims, **characterised by** the fact that it includes means (44, 56) of blocking the protector (22) in the deployed position.

12. Device according to claim 11, **characterised by** the fact that; the said means of blocking include, on the one hand, at least one tab (44) firmly attached to the support (20), and on the other hand at least one deformable hook (56), firmly attached to the protector (22); by the fact that each tab (44) and each hook (56) respectively form two effectively conjugated support surfaces, designed to deform the corresponding hook (56) in a radial direction when the support (20) and the protector (22) pass from the retracted position to the deployed position of the protector, and by the fact that each tab (44) and each hook (56) respectively form two axial and effectively connected end-stop surfaces (44B, 56B), designed to block the support (20) and the protector (22) relative to each other in the deployed position of the protector.

13. Device according to any of the preceding claims, **characterised by** the fact that it includes means of checking for the first use of the device (2).

14. Device according to claim 13, **characterised by** the fact that the said means of checking include, on the one hand, a removable cap (66) designed so that it can be arranged around the needle (6) of the syringe (1) before use, and on the other hand deformable tabs (58) arranged at the distal end of the protector (22) and which delimit a passage (60) whose diameter is, when the said tabs (58) are not deformed, less than the maximum diameter of the cap (66).

15. Injection device comprising, on the one hand, a syringe (1) which includes a needle (6), a tubular body (4) at the distal end of which is attached the needle (6) and an injection piston (8) fitted so as to slide inside the body (4), and on the other hand a device (2) for protecting the needle which is comprised of a needle protector (22), affectively coaxial with the body (4) of the syringe (1), a protector support (20) firmly attached to the said body (4) or provided with means (30) of being firmly attached to the said body (4), a spring (24) arranged supported between the support (20) and the protector (22), with the support and the protector being capable of movement relative to each other between a retracted position of the protector in which the needle (6) of the syringe (1) is exposed and in which the spring (24) is locked in the compressed state, and a deployed position in which the needle is arranged inside the protector (22) and in which the spring is, at least partially, released, and means of locking the spring (24), **characterised by** the fact that the needle protection device (2) complies with any of claims 1 to 14.

## Patentansprüche

1. Nadelschutzvorrichtung für eine Spritze (1), die eine Nadel (6), einen rohrförmigen Körper (4), an dessen distalem Ende die Nadel (6) befestigt ist, und einen Injektionskolben (8) umfasst, der in dem Körper (4) verschiebbar montiert ist, wobei diese Vorrichtung umfasst: einen zum Körper (4) der Spritze (1) im Wesentlichen koaxialen Nadelschutz (22); einen Schutzhalter (20), der mit dem Körper (4) fest verbunden ist oder mit Mitteln (30) zur festen Verbindung mit diesem Körper (4) versehen ist; eine Feder (24), die zwischen dem Halter (20) und dem Schutz (22) abgestützt angeordnet ist, wobei der Halter und der Schutz zueinander zwischen einer eingezogenen Stellung des Schutzes, in der die Nadel (6) der Spritze (1) freigelegt ist und die Feder (24) in einem komprimierten Zustand verriegelt ist, und einer ausgefahrenen Stellung beweglich sind, in der die Nadel im Inneren des Schutzes (22) angeordnet ist und die Feder mindestens teilweise entspannt ist; und Mittel zur Entriegelung der Feder (24), **dadurch gekennzeichnet, dass** die Feder (24) im verriegelten Zustand eine vorbestimmte Kraft besitzt, die größer als die Schubkraft ist, die erforderlich ist, um den Kolben (8) der Spritze (1) im Wesentlichen über seinen ganzen Injektionsweg zu bewegen, und dass die Entriegelungsmittel einen Flansch (50) zur Fingerauflage umfassen, der mit dem Schutz (22) fest verbunden ist und dafür ausgelegt ist, einerseits bei dem Verschieben des Kolbens (8) für die Injektion auf die Feder (24) eine Kompressionskraft auszuüben, die kleiner als diese vorbestimmte Kraft ist, und andererseits, wenn der Kolben (8) am Injektionswegende ist, auf die Feder (24) eine Kompressionskraft auszuüben, die größer als diese vorbestimmte Kraft ist.

2. Vorrichtung nach Anspruch 1, **dadurch gekennzeichnet, dass** die Schubkraft mindestens gleich der Summe der Injektionskraft und der Ablöse- und Gleitkräfte des Kolbens (8) im Inneren des Körpers (4) der Spritze (1) ist.

3. Vorrichtung nach einem der Ansprüche 1 oder 2, **dadurch gekennzeichnet, dass** der Flansch (50) an dem proximalen Ende des Schutzes (22) gelegen ist.

4. Vorrichtung nach einem der vorhergehenden Ansprüche, **dadurch gekennzeichnet, dass** die Länge des Schutzes (22) im Wesentlichen gleich der des Körpers (4) der Spritze (1) ist.

5. Vorrichtung nach einem der vorhergehenden Ansprüche, **dadurch gekennzeichnet, dass**, bei einer Spritze (1), deren Körper (4) an seinem proximalen Ende in fester Verbindung mit einem Hals (16) versehen ist, die Mittel zur festen Verbindung des Schutzhalters (20) mit dem Körper (4) der Spritze (1) Mittel (30) zur Befestigung des Halters an dem Hals (16) der Spritze (1) umfassen.

6. Vorrichtung nach Anspruch 5, **dadurch gekennzeichnet, dass** die Befestigungsmittel (30) mindestens einen Haken (32) zur Einhakung des Halses (16) der Spritze (1) umfassen.

7. Vorrichtung nach einem der vorhergehenden Ansprüche, **dadurch gekennzeichnet, dass** der Halter (20) und der Schutz (22) zwischen der eingezogenen Stellung des Schutzes und einer Entriegelungsstellung der Feder (24) zueinander drehbar montiert sind.

8. Vorrichtung nach Anspruch 7, **dadurch gekennzeichnet, dass** die Entriegelungsmittel einerseits mindestens eine erste Nut (40), die in einem des Halters (20) und des Schutzes (22) vorgesehen ist und mindestens zum Teil zur axialen Richtung des Körpers (4) der Spritze (1) schräg ist, und andererseits mindestens einen Zapfen (54) umfassen, der mit dem anderen des Halters (20) und des Schutzes (22) fest verbunden ist und in der ersten Nut (40) aufgenommen ist, wobei die Nut und der Zapfen zusammenwirken, um den Halter und den Schutz in Drehung zueinander zwischen der eingezogenen Stellung des Schutzes und der Entriegelungsstellung der Feder (24) zu führen.

9. Vorrichtung nach Anspruch 8, **dadurch gekennzeichnet, dass** mindestens eine zweite Nut (38) zur Aufnahme des Zapfens (54), die mit der ersten Nut (40) in Verbindung ist, in demjenigen des Halters (20) oder des Schutzes (22) vorgesehen ist, in dem die erste Nut (40) vorgesehen ist, wobei die zweite Nut (38) sich im Wesentlichen in der axialen Richtung des Körpers (4) der Spritze (1) erstreckt, so dass sie den Halter (20) und den Schutz (22) in Bezug aufeinander zwischen der Entriegelungsstellung der Feder (24) und der ausgefahrenen Stellung des Schutzes in Translation führen kann.

10. Vorrichtung nach Anspruch 9, **dadurch gekennzeichnet, dass** der distale Boden der zweiten Nut (38) für den zugeordneten Zapfen (54) einen Anschlag zur axialen Blockierung in Richtung des Ausfahrens des Schutzes (22) bildet.

11. Vorrichtung nach einem der vorhergehenden Ansprüche, **dadurch gekennzeichnet, dass** sie Mittel (44, 56) zur Blockierung des Schutzes (22) in ausgefahrener Stellung umfasst.

12. Vorrichtung nach Anspruch 11, **dadurch gekennzeichnet, dass** die Blockiermittel einerseits mindestens eine mit dem Halter (20) fest verbundene Zunge (44) und andererseits mindestens einen mit dem Schutz (22) fest verbundenen verformbaren Haken (56) umfassen, dass jede Zunge (44) und jeder Haken (56) jeweils zwei im Wesentlichen gepaarte Auflageflächen bilden, die dafür ausgelegt sind, den entsprechenden Haken (56) radial zu verformen, wenn der Halter (20) und der Schutz (22) von der eingezogenen Stellung in die ausgefahrene Stellung des Schutzes übergehen, und dass jede Zunge (44) und jeder Haken (56) jeweilig zwei im Wesentlichen gepaarte Flächen (44B, 56B) zum axialen Anschlag bildet, der dafür ausgelegt ist, den Halter (20) und den Schutz (22) in der ausgefahrenen Stellung des Schutzes zueinander zu blockieren.

13. Vorrichtung nach einem der vorhergehenden Ansprüche, **dadurch gekennzeichnet, dass** sie Mittel zur Kontrolle der ersten Verwendung der Vorrichtung (2) umfasst.

14. Vorrichtung nach Anspruch 13, **dadurch gekennzeichnet, dass** die Kontrollmittel einerseits eine lösbare Kappe (66), die dafür ausgelegt ist, vor Verwendung um die Nadel (6) der Spritze (1) angeordnet zu sein, und andererseits verformbare Zungen (58) umfassen, die am distalen Ende des Schutzes (22) vorgesehen sind und eine Durchgangsöffnung (60) begrenzen, deren Durchmesser, wenn die Zungen (58) nicht verformt sind, kleiner als der maximale Durchmesser der Kappe (66) ist.

15. Injektionsvorrichtung, umfassend einerseits eine Spritze (1), die eine Nadel (6), einen rohrförmigen Körper (4), an dessen distalem Ende die Nadel (6) befestigt ist, und einen Injektionskolben (8) umfasst, der in dem Körper (4) verschiebbar montiert ist, und andererseits eine Nadelschutzvorrichtung (2), die einen Nadelschutz (22), der zum Körper (4) der Spritze (1) im Wesentlichen koaxial ist, einen Schutzhalter (20), der mit dem Körper (4) fest verbunden ist oder mit Mitteln (30) zur festen Verbindung mit dem Körper (4) versehen ist, eine Feder (24), die zwischen dem Halter (20) und dem Schutz (22) abgestützt angeordnet ist, wobei der Halter und der Schutz zueinander zwischen einer zurückgezogenen Stellung des Schutzes, in der die Nadel (6) der Spritze (1) freigelegt ist und die Feder (24) in einem komprimierten Zustand verriegelt ist, und einer ausgefahrenen Stellung beweglich sind, in der die Nadel im Inneren des Schutzes (22) angeordnet ist und die Feder mindestens teilweise entspannt ist; und Mittel zur Entriegelung der Feder (24), **dadurch gekennzeichnet, dass** die Nadelschutzvorrichtung (2) nach einem der Ansprüche 1 bis 14 ist.
